# EUROPÄISCHE PATENTANMELDUNG

(11) **EP 4 477 238 A1**
(43) Veröffentlichungstag der Anmeldung: **18.12.2024**
(21) Anmeldenummer: 24020079.0
(22) Anmeldetag: 13.03.2024
(51) Int. Cl.: A61L 2/07, B01L 1/00, B08B 15/02

(54) **SYSTEM ZUM STERILISIEREN UND STERILEN LAGERN MEDIZINISCHER INSTRUMENTE**

(30) Priorität: 14.06.2023 CH 6332023; 14.06.2023 CH 6352023; 26.06.2023 CH 6862023; 14.07.2023 CH 7642023
(71) Anmelder: Fässler, Daniel, 5000 Aarau (CH)
(72) Erfinder: FASSLER, Daniel, 5000 Aarau (CH); HAGMANN, Peter, 4574 Nenningkofen (CH)
(74) Vertreter: Körner, Thomas Ottmar

(57) **Zusammenfassung**

System zum Sterilisieren und zum sterilen Lagern und/oder Aufbewahren medizinischer Instrumente, welchem zu sterilisierende medizinische, insbesondere zahnmedizinische, Instrumente als Sterilisationsgut zugeführt und sterilisierte medizinische Instrumente als Sterilgut entnommen werden können, besagtes System umfassend: einen Sterilisator (1), insbesondere einen Autoklaven, umfassend:
einen ersten Innnenraum (11) zur Aufnahme von Sterilisationsgut,
eine Entladeöffnung (12) zum Entnehmen von Sterilgut aus dem ersten Innenraum,
eine Entladeklappe (121), mittels welcher die Entladeöffnung fluid- und druckdicht verschlossen werden kann;
eine Sicherheitswerkbank (2), insbesondere eine Laminar Flow Box, umfassend:
einen zweiten Innenraum (21), insbesondere zur Aufbewahrung von Sterilgut,
eine Befüllungsöffnung (23) zur Befüllung des zweiten Innenraums mit Sterilgut,
eine Entnahmeöffnung zur Entnahme von Sterilgut aus dem zweiten Innenraum; dadurch gekennzeichnet, dass
die Entladeöffnung in die Befüllungsöffnung mündet.

## Beschreibung

Die Erfindung bezieht sich auf eine für eine Vorbereitung und Handhabung medizinsicher, insbesondere zahnmedizinischer, Instrumente notwendige Logistik. Sie betrifft ein System und Verfahren zum Sterilisieren und sterilen Aufbewahren und/oder Lagern medizinischer Instrumente sowie ein Verfahren zum Bereitstellen eines entsprechenden Systems gemäss den unabhängigen Patentansprüchen.

### Hintergrund der Erfindung

Durch die neuen Hygienevorschriften für Zahnarztpraxen oder Ähnliches (z.B. Podologie) entsteht viel mehr Arbeit und Abfall.

Insbesondere in der zahnärztlichen Praxis können Instrumente und Werkzeuge hinsichtlich hygienischer Anforderungen in drei Klassen eingeteilt werden: kritische Instrumente bzw. Werkzeuge, halbkritische Instrumente bzw. Werkzeuge, und nichtkritische Instrumente bzw. Werkzeuge.

Insbesondere jedes kritische Werkzeug, Instrument etc. muss vor dem Einbringen in einen Sterilisator, insbesondere einen Autoklaven, in welchem eine Sterilisation von Instrumenten und Werkzeugen in der (zahn-)ärtzlichen Praxis in der Regel erfolgt, in einen Beutel, insbesondere einen sogenannten Sterilisationsbeutel, eingeschweisst und vor dessen Gebrauch, insbesondere erst unmittelbar vor Gebrauch, wieder ausgepackt werden.

Alles, was eingepackt werden muss, hat auch mehr Volumen und erschwert eine Übersicht in der Lagerhaltung in der Praxis. Die Sicht zu den Werkzeugen etc. ist viel eingeschränkter. Zudem entsteht eine Abhängigkeit von einer Verfügbarkeit und/oder zu den Lieferanten der Beutel.

Durch das erhöhte Volumen wird eine Kapazität eines Sterilisationsprozesses bzw. eines vorhandenen Sterilisators reduziert, insbesondere können pro Sterilisationsprozess und somit pro Zeiteinheit eine wesentlich geringere Anzahl Werkzeuge, Instrumente etc. sterilisiert werden, als dies bei einer Sterilisation in nicht eingeschweisstem Zustand der Fall wäre. Dies ist auch darauf zurückzuführen, dass Sterilisationsbeutel mit eingeschweissten Werkzeugen, Instrumenten etc. mit einem grösseren Abstand zueinander im Autoklaven positioniert werden müssen als dies bei nicht eingeschweissten Werkzeugen, Instrumenten etc. der Fall wäre, da ansonsten ein unbefriedigendes Sterilisationsergebnis resultieren kann.

Auch in eingeschweisstem Zustand, d.h. im Sterilisationsbeutel, können kritische Werkzeuge, Instrumente etc. jedoch nicht zeitlich unbegrenzt aufbewahrt werden. Dies liegt Daran, dass die Sterilisationsbeutel im Laufe der Zeit undicht werden, was insbesondere auf kleinere, teilweise mit blossem Auge nicht sichtbare Beschädigungen wie Löcher oder Schnitte zurückzuführen sein kann. Diese können mehr oder weniger zwangsläufig entstehen, wenn die Sterilisationsbeutel mit den grösstenteils spitzen und oder scharfen Werkzeuge, Instrumente etc. behändigt oder anderweitig bewegt werden, insbesondere beim Herausnehmen einzelner Sterilisationsbeutel aus einer Ansammlung z.B. in einem Sortimentskasten oder einer Sortimentsbox, bei der Lagerung in Schubladen bei deren Öffnen und Schliessen, usw. Teilweise werden die Sterilisationsbeutel, auch mit Blick auf die vorstehend geschilderten Probleme, mit einem Verfalldatum versehen, nach welchem die enthaltenen Werkzeuge, Instrumente etc. nicht mehr verwendet werden dürfen.

Wegen einer eingeschränkten Sichtbarkeit der Werkzeuge, Instrumente etc. im Sterilisationsbeutel kommt es auch immer wieder vor, dass versehentlich ein falsches, insbesondere für einen beabsichtigten Verwendungszweck ungeeignetes oder zumindest suboptimales Werkzeug, Instrument etc. ausgepackt wird.

In den vorgenannten Fällen müssen die Werkzeuge, Instrumente etc. erneut eingeschweisst und einem Sterilisationsprozess unterzogen werden, bevor sie erneut verwendet werden können. Dies führt zu einem an sich unnötigen Bedarf an Ressourcen, einerseits in Form von Rohstoffen wie für die Herstellung der Sterilisationsbeutel benötigtem Kunststoff und somit letztendlich Erdöl, andererseits in Form von Energie sowohl für die Herstellung der Sterilisationsbeutel als auch für den Sterilisationsprozess selbst. Ferner entsteht unnötiger Abfall in Form zusätzlich benötigter Sterilisationsbeutel. Schliesslich wird Arbeitskraft gebunden, um beschädigte und/oder abgelaufene Sterilisationsbeutel auszusortieren, und die nicht zum Einsatz gekommen Werkzeuge, Instrumente etc. erneut einzuschweissen und zu sterilisieren.

Der Erfindung liegt daher die Aufgabe zugrunde, ein System und ein Verfahren zum Sterilisieren und sterilen Lagern medizinischer Instrumente und Werkzeuge anzugeben, welche die vorstehend geschilderten Nachteile vermeidet oder vermindert, sowie ein Verfahren zum Bereitstellen eines entsprechenden Systems.

### Kurze Beschreibung der Erfindung

Diese Aufgabe kann erfindungsgemäss durch ein System und Verfahren zum Sterilisieren und sterilen Lagern medizinischer Instrumente und/oder Werkzeuge sowie ein Verfahren zum Bereitstellen eines entsprechenden Systems gemäss den unabhängigen Patentansprüchen gelöst werden.

In den nachfolgenden Ausführungen, insbesondere den Patentansprüchen, wird der Begriff Instrumente sowohl für Instrumente als auch für Werkzeuge verwendet.

System und Verfahren beruhen auf der Idee, Instrumente, welche vor einem Einsatz im Rahmen einer Behandlung eines Patienten sterilisiert werden müssen, und insbesondere bis unmittelbar vor diesem Einsatz in sterilem Zustand gehalten werden müssen, in unverpacktem, insbesondere nicht eingeschweissten Zustand in einem Sterilisator, insbesondere einem ersten Innenraum eines Sterilisators, zu sterilisieren, und nach erfolgter Sterilisation direkt und unmittelbar in eine Sicherheitswerkbank, insbesondere einen zweiten Innenraum einer Sicherheitswerkbank, zu verbringen, und diese dort bis unmittelbar vor ihrem Einsatz zu lagern. Um dies zu ermöglichen, kann an der Sicherheitswerkbank, bei der es sich insbesondere um eine Laminar Flow Box handeln kann, zusätzlich zu einer herkömmlichen Arbeitsöffnung eine separate Befüllungsöffnung vorgesehen sein. Eine Entladeöffnung des Sterilisators, welche insbesondere bei einem einfachen, herkömmlichen und/oder handelsüblichen Sterilisator auch der Beladung dienen kann. Die Entladeöffnung kann dabei derart mit der Befüllungsöffnung verbunden sein oder werden, dass die Entladeöffnung in die Befüllungsöffnung mündet und/oder geführt ist, insbesondere in die Befüllungsöffnung hineinragt. Dies erlaubt es, sterilisierte Instrumente dem Sterilisator zu entnehmen und in den zweiten Innenraum der Sicherheitswerkbank zu verbringen, ohne dass diese eine nicht sterile Umgebung durchqueren müssen, in welcher Sterilisator uns Sicherheitswerkbank aufgestellt sind.

Zu sterilisierende Instrumente, insbesondere Instrumente, die im Rahmen einer Behandlung eines Patienten eingesetzt wurden, können nachfolgend als Sterilisationsgut bezeichnet werden. Nach erfolgter Sterilisation sind diese erneut einsatzbereit, und werden als Sterilgut bezeichnet.

Ein System zum Sterilisieren und sterilen Lagern medizinischer Instrumente gemäss der Erfindung wie nachfolgend beansprucht kann die Merkmale von Anspruch 1 weiter unten umfassen.

Ein erfindungsgemässes System zum Sterilisieren und zum sterilen Lagern und/oder Aufbewahren medizinischer Instrumente, welchem zu sterilisierende medizinische, insbesondere zahnmedizinische, Instrumente als Sterilisationsgut zugeführt und sterilisierte medizinische Instrumente als Sterilgut entnommen werden können, kann insbesondere umfassen:
a. einen Sterilisator (1), insbesondere einen Autoklaven, umfassend:
   i. einen ersten Innnenraum (11) zur Aufnahme von Sterilisationsgut,
   ii. eine Entladeöffnung (12) zum Entnehmen von Sterilgut aus dem ersten Innenraum,
   iii. eine Entladeklappe (121), mittels welcher insbesondere die Entladeöffnung fluid- und druckdicht verschlossen werden kann;
b. eine Sicherheitswerkbank (2), insbesondere eine Laminar Flow Box, umfassend:
   i. einen zweiten Innenraum (21), insbesondere zur Aufbewahrung von Sterilgut,
   ii. eine Befüllungsöffnung (23) zur Befüllung des zweiten Innenraums mit Sterilgut,
   iii. eine Entnahmeöffnung zur Entnahme von Sterilgut aus dem zweiten Innenraum; wobei
   c. die Entladeöffnung mit der Befüllungsöffnung verbunden ist, insbesondere in die Befüllungsöffnung mündet und/oder geführt ist, insbesondere in die Befüllungsöffnung hineinragt.

Der Sterilisator kann dazu ausgelegt sein, in bzw. während eines Sterilisationsprozesses oder -programms in seinem ersten Innenraum Bedingungen einzustellen, die zumindest mit an Sicherheit grenzender Wahrscheinlichkeit Organismen, Keime, Bakterien, Viren, Sporen, Prionen und/oder weitere Krankheitserreger, welche sich ersten Innenraum und/oder auf in diesem befindlichen Instrumenten abtöten und/oder zerstören, insbesondere während einer ersten Zeitspanne, welche kürzer ist als eine zweite Zeitspanne von einem Beginn bis zu einem Ende des Sterilisationsprozesses oder -programms. Die genannten Bedingungen können dabei insbesondere eine Temperatur oberhalb von 100°C umfassen, insbesondere von oder oberhalb von 121°C.

Beim Sterilisator kann es sich dabei insbesondere um eine Vorrichtung handeln, mittels welcher eine Sterilisation, insbesondere ein Sterilisationsprozess, wie in den Wikipedia Artikeln https://en.wikipedia.org/w/index.php?title=Sterilization (microbiology)&ol-did=1158241630
und
https://de.wikipedia.org/w/index.php?title=Sterilisation&oldid=234122836 beschrieben, durchgeführt werden kann; insbesondere um einen Autoklaven wie in den Wikipedia Artikeln
https://de.wikipedia.org/w/index.php?title=Autoklav&oldid=229184556
   und
https://en.wikipedia.org/w/index.php?title=Autoclave&oldid=1145034987 beschrieben. Insbesondere kann es sich beim Autoklaven um einen herkömmlichen und/oder handelsüblichen Autoklaven handeln, wie er in kleineren ärztlichen oder zahnärztlichen Praxen, insbesondere mit maximal 5 Ärzten bzw. Zahnärzten zum Einsatz kommt. Ein derartiger Autoklav weist in der Regel eine einzige Öffnung auf, durch welche er mit Sterilisationsgut beladen werden kann, und durch welche nach erfolgter Sterilisation das Sterilgut entnommen werden kann. Die einzige Öffnung kann somit als Entladeöffnung bezeichnet werden.

Bei der Sicherheitswerkbank kann es sich dabei insbesondere um eine Vorrichtung handeln, wie in den Wikipedia Artikeln
https://de.wikipedia.org/w/index.php?title=Sicherheitswerkbank&oldid=227074093 und
https://en.wikipedia.org/w/index.php?title=Laminar_flow_cabinet&oldid=1142649438 beschrieben.

Im zweiten Innenraum der Sicherheitswerkbank können dabei insbesondere eine Reinraumatmosphäre und/oder sterile Bedingungen herrschen und/oder aufrechterhalten werden, insbesondere Bedingungen gemäss Reinraumklasse ISO 14644-1.

Als Entnahmeöffnung kann insbesondere eine Arbeitsöffnung der Sicherheitswerkbank dienen, durch welche Bedienpersonal, insbesondere eine Praxisassistenz oder ein(e) Dentalhygieniker(in) in den zweiten Innenraum hineingreifen und dort Manipulationen vornehmen kann. Die Arbeitsöffnung kann mittels einer Klappe, Scheibe oder Ähnlichem verschliessbar sein.

Da die Entladeöffnung wie vorstehend beschrieben mit der Befüllungsöffnung verbunden ist, kann das Bedienpersonal Sterilisationsgut durch den zweiten Innenraum, die Befüllungsöffnung und die Entladeöffnung in den Sterilisator einbringen anschliessend die Entladeöffnung mittels der Endladeklappe verschliessen, und das Sterilisationsprogramm starten. Nach dem Ende des Sterilisationsprogramms kann das Bedienpersonal das Sterilgut durch die Entladeöffnung und die Befüllungsöffnung entnehmen und innerhalb des zweiten Innenraums platzieren, wozu insbesondere Ablagen in Form von Tablaren, Trays, Sortimentskästen oder -boxen, etc. vorgesehen sein können.

Zusätzlich zur Entladeöffnung kann der Sterilisator eine separate Beladeöffnung zum Einbringen von Sterilisationsgut in den ersten Innenraum aufweisen sowie eine Beladeklappe (131), mittels welcher die Beladeöffnung fluid- und druckdicht verschlossen werden kann. Die Beladeöffnung kann von der Entladeöffnung entfernt angeordnet sein, insbesondere an einer anderen, insbesondere gegenüberliegenden Seite des Sterilisators. Durch die separate Beladeöffnung entfällt die Notwendigkeit, Sterilisationsgut durch den zweiten Innenraum, in den Sterilisator, insbesondere den ersten Innenraum, einbringen zu müssen, wie dies bei einem Autoklaven mit einer einzigen Öffnung erforderlich ist. Damit kann eine Sicherheit der sterilen Lagerung des Sterilguts im zweiten Innenraum weiter erhöht werden. Ferner kann eine Handhabung des Systems erleichtert und/oder beschleunigt werden.

Bei einem erfindungsgemässen System kann ein Verriegelungsmechanismus vorgesehen sein, welcher dazu eingerichtet sein kann, die Entladeklappe nach einem Verschliessen zu verriegeln, so dass diese erst nach einem Ende, insbesondere einem regulären Ende, des Sterilisationsprozess wieder geöffnet werden kann. Dies verhindert, dass versehentlich Verunreinigungen in den zweiten Innenraum gelangen können, und eine dort herrschende Sterilität und/oder Raumreinbedingungen komprimieren und/oder zunichte machen kann. Ohne einen entsprechenden Verriegelungsmechanismus kann dies insbesondere durch versehentliche Entnahme und ggf. Einlagerung nicht (ausreichend) sterilisierter Instrumente geschehen.

Bei einem System mit einem Sterilisator mit einer separaten Beladeöffnung kann der Verriegelungsmechanismus zusätzlich oder alternativ dazu eingerichtet sein, ein Öffnen der Entladeklappe zu verhindern, solange die Beladeklappe geöffnet ist, und/oder ein Öffnen der Beladeklappe zu verhindern, solange die Entladeklappe geöffnet ist. Dadurch kann verhindert werden, dass Be- und Entladeklappe gleichzeitig geöffnet sind und dadurch Verunreinigungen durch den ersten in den zweiten Innenraum gelangen können, womit wiederum eine dort herrschende Sterilität und/oder Raumreinbedingungen komprimiert und/oder zunichte gemacht werden könnte.

Ein Verriegelungsmechanismus wie vorstehend beschrieben erlaubt insbesondere eine prozesssichere Überwachung von Be- und/oder Entladeklappe.

Ein Verfahren zum Bereitstellen eines Systems zum Sterilisieren und zum sterilen Lagern und/oder Aufbewahren medizinischer Instrumente wie nachfolgend beansprucht kann die Merkmale von Anspruch 2 weiter unten umfassen.

Für das Verfahren gelten die weiter oben gemachten Ausführungen, insbesondere betreffend den Sterilisator und die Sicherheitswerkbank, analog.

Vorteilhafte Ausgestaltungen der Erfindung sind Gegenstand der Unteransprüche, und/oder ergeben sich aus der nachfolgenden Beschreibung in Verbindung mit den Zeichnungen.

Im Folgenden wird anhand beiliegender Zeichnungen ein bevorzugtes Ausführungsbeispiel der Erfindung beschrieben.

Es zeigen
- Figur 1: schematisch einen grundsätzlichen Aufbau eines erfindungsgemässen Systems zum Sterilisieren und sterilen Lagern medizinischer Instrumente;
- Figuren 2a-c: Details betreffend die Sicherheitswerkbank.

### Wege zur Ausführung der Erfindung

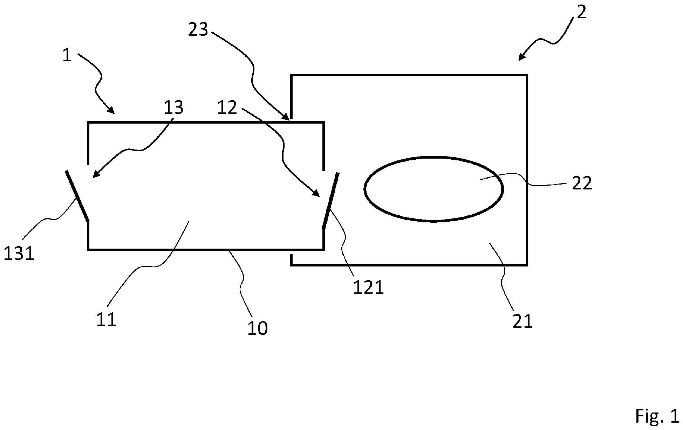

Figur 1 zeigt eine schematische Darstellung eines erfindungsgemässen Systems und illustriert das erfindungsgemässe Bereitstellungsverfahren. Definitionen der Bezugszeichen finden sich in den Patentansprüchen, wo diese in Klammern angegeben sind. Ein Teil des Sterilisators 1 mit einem ersten Innenraum 11 ist bzw. wird durch eine Befüllungsöffnung 23 einer Sicherheitswerkbank 2 teilweise in einen zweiten Innenraum 21 besagter Sicherheitswerkbank eingeschoben, so dass eine Entladeöffnung 12 des Sterilisators in die Befüllungsöffnung mündet, insbesondere in die Befüllungsöffnung hineinragt. Eine erste Form der Befüllungsöffnung 23 ist dabei vorzugsweise so auf eine zweite Form eines Gehäuses 10 des Sterilisators abgestimmt, dass zwischen besagtem Gehäuse und einem Rand der Befüllungsöffnung 23 lediglich ein schmaler Spalt verbleibt, dessen Breite vorzugsweise an keiner Stelle grösser ist als wenige Millimeter. Der Spalt kann vorzugsweise fluiddicht abgedichtet sein oder werden, insbesondere mittels einer Silikonfuge oder einer Gummidichtung. Durch den schmalen Spalt und insbesondere eine Abdichtung kann verhindert werden, dass Verunreinigungen durch die Befüllungsöffnung in denzweiten Innenraum gelangen können, womit eine dort herrschende Sterilität und/oder Raumreinbedingungen komprimiert und/oder zunichte gemacht werden könnte.

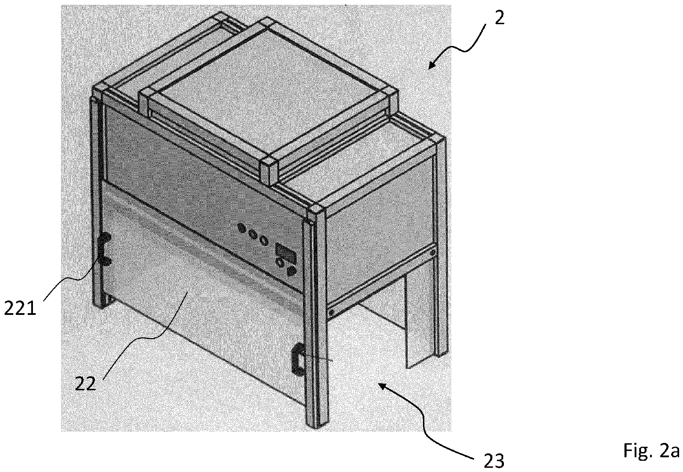

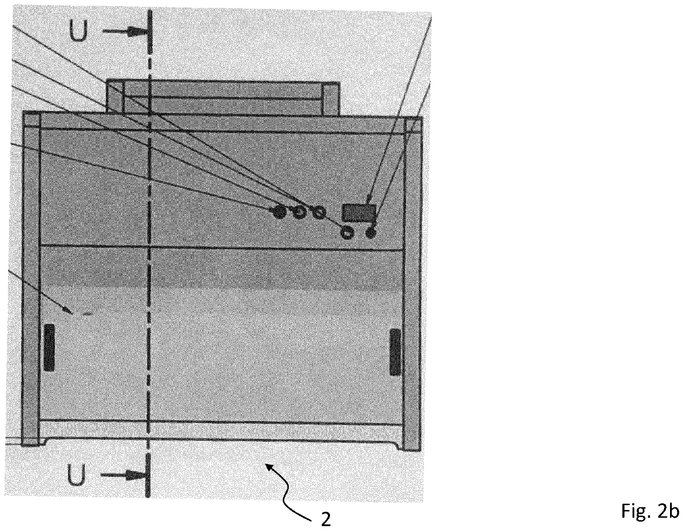

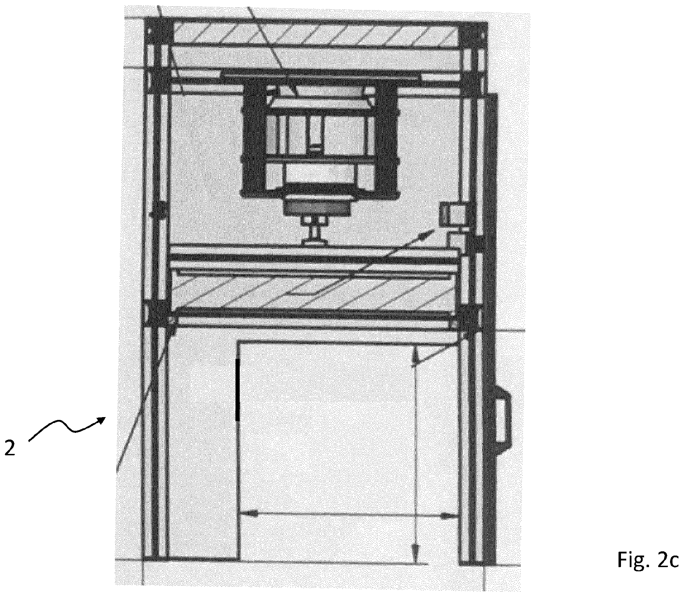

Die Figuren 2a bis 2c zeigen Details einer exemplarischen Sicherheitswerkbank 2 zur Verwendung in einem System oder Verfahren wie vorstehend beschrieben und oder nachfolgend beansprucht. In die Sicherheitswerkbank in Form einer Laminarflow-Kabine (LA) gebaut nach Reinraumklassen ISO 14644-1 wird seitlich durch eine Aussparung, welche als Befüllungsöffnung 23 dient, ein Autoklav von der Tür-Seite her, insbesondere mit derjenigen Seite, an welcher die Entladeklappe vorgesehen ist, zumindest annähernd 10 cm weit eingeschoben und bleibt so installiert. Die Masse des, für diesen Fall, vorgesehen Autoklavs sind zumindest annähernd B × H × L 450 × 440 × 620 mm. Alternativ wäre es auch möglich, die Befüllungsöffnung an einer hinteren Wand der LA vorzusehen und entsprechend den Autoklaven von der hinteren Wand her einzubauen, dies erfordert jedoch mehr Tiefe für eine Aufstellung eines entsprechenden Systems.

Sobald der Entkeimungsvorgang, insbesondere der Sterilisationsprozess, beendet ist und sich die Türe des Autoklavs automatisch öffnet, bleibt der Inhalt in Reinraumatmosphäre.

Das Personal kann dann nach Anweisung des Arztes den Inhalt, also die Werkzeuge und Instrumente etc. in die Trays für den Arzt oder DA einordnen und unter Reinraumbedingungen so lange lagern, bis ein Patient auf dem Stuhl sitzt. Dann werden die Trays mit einem Deckel an den Arbeitsplatz gebracht. Der Deckel wird erst entfernt, wenn die Werkzeuge oder die Instrumente etc. gebraucht werden. Sobald die Behandlung abgeschlossen ist, werden die Instrumente etc. mit dem Tray und dem Deckel in die Reinigung zurückgestellt, insbesondere eine sog. rote Zone, in welcher Instrumente aufbewahrt werden, die vor einem neuerlichen Einsatz zunächst sterilisiert werden müssen.

Nach der Reinigung der Instrumente etc. beginnt der Prozess von Neuem.

Alle Teile, wie die Lagergestelle, die Trays und deren Deckel, welche sich unter dem LA befinden, lassen sich im Autoklav entkeimen. Das soll periodisch gemäss dem Ausdruck vom Autoklav oder nach Anweisung des Arztes durchgeführt werden.

Die jetzige Lösung soll sich für Praxen bis zwei Zahnarztplätze und bis zu zwei DH-Arbeitsplätze eignen.

Es sind 3 x 7 Stapelplätze für das Tray Format mit Deckel B × H × L 186 × 45 × 288 mm vorgesehen.

Eine Arbeitsöffnung 22 der LA ist mittels einer Frontscheibe 221 verschliessbar, welche insbesondere vertikal verschiebbar sein kann, um die Arbeitsöffnung je nach Position besagter Frontscheibe freizugeben oder zu verschliessen.

Die exemplarische Sicherheitswerkbank weist einen G4-Vorfilter auf, der insbesondere der Prüfnorm ISO 16890 entspricht mit einer Filterklasse ISO coarse 85% / G4; ferner einen H1-Schwebstoffiler, der insbesondere der Prüfnorm EN 1822 entspricht, insbesondere mit einer Filterklasse H14, einem minimalen Abscheidegrad von zumindest annähernd 99,995% und/oder einem maximalen Durchlassgrad von zumindest annähernd 0,00%.

Im ersten Innenraum der Sicherheitswerkbank kann eine Beleuchtung vorgesehen sein, welche insbesondere LEDs umfassen kann.

Für grössere Praxen können die Dimensionen des LA und ev. des Autoklavs sowie die Stapelplätze angepasst werden.

Weiter ist ein Autoklav denkbar, welcher sich von aussen beladen lässt, insbesondere über eine separate Beladeöffnung, und sich dieser nach dem Sterilisierungsvorgang innen öffnet. Beide Türen müssen prozesssicher überwacht werden. Das hat den Vorteil, dass nie unsteriles Material in die Reinraumatmosphäre gelangt. Zudem ist das Handling einfacher.

Sofern nicht anders angegeben, kann ein fluid- und/oder druckdichtes Element, insbesondere ein Behälter, ein Volumen oder ein Rückhaltevolumen, ein Fluid, insbesondere eine Flüssigkeit oder ein Gas, speichern, enthalten, vorrätig halten und/oder bevorraten, ohne dass das Fluid aus besagtem Element austritt oder austreten kann. Sofern nicht anders angegeben, kann eine fluid- und/oder druckdichte Verbindung zwischen zwei ein Fluid, insbesondere eine Flüssigkeit oder ein Gas, leitenden, führenden, bevorratenden und/oder speichernden Elementen wie insbesondere Leitungen, Rohren, Behältern, Volumen, Rückhaltevolumen, etc., eine Verbindung bezeichnen, durch welche Fluid verlustfrei, insbesondere ohne durch die oder im Bereich der Verbindung auszutreten, von einem Element zum anderen strömen und/oder geleitet werden kann, insbesondere unter den üblicherweise, vor allem in einem Betriebszustand einer die Elemente umfassenden Apparatur, herrschenden, statischen und/oder dynamischen Fluiddrücken. In analoger Weise kann ein fluid- und/oder druckdichter Verschluss oder eine fluid- und/oder druckdichte (Ab-)dichtung ein Element, insbesondere eine Öffnung eines Elements, verschliessen, ohne dass Fluid durch den oder im Bereich des Verschlusses bzw. der (Ab-)dichtung austreten kann. Eine Fluidverbindung kann insbesondere eine fluid- und/oder druckdichte Verbindung sein. Aus einem oder in ein fluid- und/oder druckdicht verschlossenes Volumen, insbesondere aus einem oder in einen fluid- und/oder druckdicht verschlossenen Behälter, kann. Ein druckdichtes Element, eine druckdichte Verbindung oder ein druckdichter Verschluss bleibt dabei auch unter einer wesentlichen Druckdifferenz, insbesondere zwischen einem Inneren und einem Äusseren des Elements oder der Verbindung oder beiden Seiten des Verschlusses, fluiddicht. Eine wesentliche Druckdifferenz kann mindestens einen Faktor 1,5, 2,0 oder 5,0 bedeuten, wenn sich sowohl im Inneren als auch dem Äusseren des Elements oder der Verbindung oder auf beiden Seiten des Verschlusses ein gasförmiges Fluid vorliegt. Eine wesentliche Druckdifferenz kann mindestens einen Faktor 10, 50 oder 200 bedeuten, wenn im Inneren oder Äusseren des Elements oder der Verbindung oder auf mindestens einer Seiten des Verschlusses ein Fluid in Form einer Flüssigkeit vorliegt.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Um die Erfindung nicht zu verklären, können in gewissen Fällen wohlbekannte Strukturen und Techniken nicht im Detail gezeigt und beschrieben sein. Es versteht sich, dass Fachleute Änderungen und Abwandlungen machen können, ohne den Umfang der folgenden Ansprüche zu verlassen. Insbesondere deckt die vorliegende Erfindung weitere Ausführungsbeispiele mit irgendwelchen Kombinationen von Merkmalen ab, die von den explizit beschriebenen Merkmalskombinationen abweichen können.

Die vorliegende Offenbarung umfasst auch Ausführungsformen mit jeglicher Kombination von Merkmalen, die vorstehend oder nachfolgend zu verschiedenen Ausführungsformen genannt oder gezeigt sind. Sie umfasst ebenfalls einzelne Merkmale in den Figuren, auch wenn sie dort im Zusammenhang mit anderen Merkmalen gezeigt sind und/oder vorstehend oder nachfolgend nicht genannt sind. Auch können die in den Figuren und der Beschreibung beschriebenen Alternativen von Ausführungsformen und einzelne Alternativen deren Merkmale vom Erfindungsgegenstand beziehungsweise von den offenbarten Gegenständen ausgeschlossen sein. Die Offenbarung umfasst Ausführungsformen, die ausschliesslich die in den Ansprüchen beziehungsweise in den Ausführungsbeispielen beschriebenen Merkmale umfasst sowie auch solche, die zusätzliche andere Merkmale umfassen.

Im Weiteren schliesst der Ausdruck "umfassen" und oder Ableitungen davon andere Elemente oder Schritte nicht aus. Ebenfalls schliesst der unbestimmte Artikel "ein" bzw. "eine" und Ableitungen davon eine Vielzahl nicht aus. Die Funktionen mehrerer in den Ansprüchen aufgeführter Merkmale können durch eine Einheit beziehungsweise einen Schritt erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Die Begriffe "etwa" und "ungefähr" im Zusammenhang mit einem gegebenen Zahlenwert oder -bereich kann sich auf einen Wert beziehungsweise Bereich beziehen, der innerhalb 20%, innerhalb 10%, innerhalb 5% oder innerhalb 2% des gegebenen Werts beziehungsweise Bereichs liegt. Alle Bezugszeichen in den Ansprüchen sind nicht als den Umfang der Ansprüche einschränkend zu verstehen. Eine Angabe, wonach *a* ≈ *b* gilt, kann dahingehend zu verstehen sein, dass |*a*-*b*|/(|*a*|+|*b*|) < 0,2, vorzugsweise |*a*-*b*|/(|*a*|+|*b*|) < 0,05, höchst vorzugsweise |*a*-*b*|/(|*a*|+|*b*|) < 0,01 gilt, wobei a und b beliebige, an irgendeiner Stelle in diesem Dokument definierte und/oder beschriebene oder anderweitig dem Fachmann bekannte Variablen oder Grössen repräsentieren kann. Der Ausdruck «einige» kann eine Anzahl zwischen 3 und 10, vorzugsweise zwischen 5 und 9 bezeichnen.

Dass ein Merkmal oder eine Eigenschaft, beispielsweise eine spezifische, insbesondere geometrische, Form, zumindest näherungsweise ausgebildet, vorgesehen oder vorhanden ist, kann insbesondere bedeuten, dass Fertigungsvorgaben existieren, welche eine Vorgabe vorsehen, gemäss welcher das Merkmal entsprechend ausgebildet wird, wobei im Rahmen üblicher, dem Fachmann bekannter Fertigungstoleranzen eine Abweichung von der Vorgabe resultieren kann.

Dass ein Element oder Merkmal in einer Richtung ausgedehnt ist oder sich in einer Richtung erstreckt, kann insbesondere bedeuten, dass Abmessungen des Elements oder Merkmals in dieser Richtung grösser sind als in anderen, insbesondere allen anderen Richtungen, insbesondere orthogonalen Richtungen.

Die Begriffe "oben", "unten", "vorne", "hinten" beziehen sich insbesondere auf die bzw. den Aufbau des erfindungsmässen Systems in derjenigen Orientierung, in welcher dieses in Fig. 1 gezeigt ist. Dabei verläuft die *y*-Richtung von «oben» nach «unten». Eine seitliche Richtung entspricht der *z*-Richtung (wie in Fig. 1 angezeigt senkrecht zur Zeichenebene in diese hinein) oder -*z*-Richtung.

Sämtliche oben in Bezug genommenen Dokumente, insbesondere Wikipedia-Artikel, gelten als in vollem Umfang per Verweisung inkludiert.

## Patentansprüche

1. System zum Sterilisieren und zum sterilen Lagern und/oder Aufbewahren medizinischer Instrumente, welchem zu sterilisierende medizinische, insbesondere zahnmedizinische, Instrumente als Sterilisationsgut zugeführt und sterilisierte medizinische Instrumente als Sterilgut entnommen werden können, besagtes System umfassend:
a. einen Sterilisator (1), insbesondere einen Autoklaven, umfassend:
i. einen ersten Innnenraum (11) zur Aufnahme von Sterilisationsgut,
ii. eine Entladeöffnung (12) zum Entnehmen von Sterilgut aus dem ersten Innenraum,
iii. eine Entladeklappe (121), mittels welcher insbesondere die Entladeöffnung fluid- und druckdicht verschlossen werden kann;
b. eine Sicherheitswerkbank (2), insbesondere eine Laminar Flow Box, umfassend:
i. einen zweiten Innenraum (21), insbesondere zur Aufbewahrung von Sterilgut,
ii. eine Befüllungsöffnung (23) zur Befüllung des zweiten Innenraums mit Sterilgut,
iii. eine Entnahmeöffnung zur Entnahme von Sterilgut aus dem zweiten Innenraum;
**dadurch gekennzeichnet, dass**
c. die Entladeöffnung mit der Befüllungsöffnung verbunden ist, insbesondere in die Befüllungsöffnung mündet und/oder geführt ist, insbesondere in die Befüllungsöffnung hineinragt.

2. Verfahren zum Bereitstellen eines Systems zum Sterilisieren und zum sterilen Lagern und/oder Aufbewahren medizinischer Instrumente, umfassend die Schritte:
a. Bereitstellen eines Sterilisators (1), insbesondere einen Autoklaven, umfassend:
i. einen ersten Innnenraum (11) zur Aufnahme von Sterilisationsgut,
ii. eine Entladeöffnung (12) zum Entnehmen von Sterilgut aus dem ersten Innenraum,
iii. eine Entladeklappe (121), mittels welcher insbesondere die Entladeöffnung fluid- und druckdicht verschlossen werden kann;
b. Bereitstellen einer Sicherheitswerkbank (2), insbesondere einer Laminar Flow Box, umfassend:
i. einen zweiten Innenraum (21), insbesondere zur Aufbewahrung von Sterilgut,
ii. eine Befüllungsöffnung (23) zur Befüllung des zweiten Innenraums mit Sterilgut,
iii. eine Entnahmeöffnung zur Entnahme von Sterilgut aus dem zweiten Innenraum;
**gekennzeichnet durch**
c. Verbinden der Entladeöffnung mit der Befüllungsöffnung, insbesondere derart, dass die Entladeöffnung in die Befüllungsöffnung mündet und/oder geführt ist, insbesondere in die Befüllungsöffnung hineinragt.

3. System oder Verfahren nach Anspruch 1 oder 2, **dadurch gekennzeichnet, dass** der Sterilisator ferner aufweist:
a. eine Beladeöffnung (13) zum Einbringen von Sterilisationsgut in den ersten Innenraum und
b. eine Beladeklappe (131), mittels welcher die Beladeöffnung fluid- und druckdicht verschlossen werden kann, wobei
c. die Beladeöffnung von der Entladeöffnung entfernt angeordnet ist, vorzugsweise an einer anderen, insbesondere gegenüberliegenden Seite des Sterilisators.

4. System oder Verfahren nach einem der vorangehenden Ansprüche, **dadurch gekennzeichnet, dass** die Entladeöffnung fluiddicht mit der Befüllungsöffnung verbunden ist bzw. wird, und/oder eine fluiddichte Verbindung zwischen der Entladeöffnung und der Befüllungsöffnung vorgesehen ist bzw. wird.

5. System oder Verfahren nach einem der vorangehenden Ansprüche, **dadurch gekennzeichnet, dass** die Entnahmeöffnung eine Arbeitsöffnung (22) ist, durch welche insbesondere
a. Bedienpersonal in den zweiten Innenraum greifen kann, insbesondere um im zweiten Innenraum befindliches Sterilgut zu entnehmen oder innerhalb des zweiten Innenraums zu bewegen, insbesondere zu verschieben oder umzuplatzieren;
b. durch den ersten Innenraum und die Entladeöffnung in den ersten Innenraum greifen kann, insbesondere um Sterilgut aus dem ersten Innenraum zu entnehmen und durch die Entladeöffnung in den zweiten Innenraum zu verbringen; wobei
c. die Arbeitsöffnung vorzugsweise verschliessbar ist, insbesondere mittels einer Abdeckung, welche insbesondere klappbar oder verschiebbar ausgeführt ist.

6. System oder Verfahren nach einem der vorangehenden Ansprüche, **dadurch gekennzeichnet, dass** ein Verriegelungsmechanismus vorgesehen ist bzw. wird, welcher dazu eingerichtet ist, die Entladeklappe nach einem Verschliessen zu verriegeln, so dass diese erst wieder geöffnet werden kann, nachdem ein Sterilisationsprozess durchgeführt wurde.

7. System oder Verfahren nach Anspruch 6, **dadurch gekennzeichnet, dass** der Verriegelungsmechanismus dazu eingerichtet ist, ein Öffnen der Entladeklappe zu verhindern, solange die Beladeklappe geöffnet ist, und/oder ein Öffnen der Beladeklappe zu verhindern, solange die Entladeklappe geöffnet ist.

8. System oder Verfahren nach einem der vorangehenden Ansprüche, **dadurch gekennzeichnet, dass** die Befüllungsöffnung und die Entnahmeöffnung an unterschiedlichen Seiten der Sicherheitswerkbank vorgesehen sind.
